# EUROPEAN PATENT APPLICATION

(11) **EP 2 363 163 A1**
(43) Date of publication of application: **07.09.2011**
(21) Application number: 10013762.9
(22) Date of filing: 20.03.2009
(51) Int. Cl.: A61M 16/12

(54) **Device for controlled delivery of breathing gas to a patient using multiple ventilation parameters**

(30) Priority: 27.03.2008 US 40089 P
(62) Divisional of application: 09724401.6
(71) Applicant: Nellcor Puritan Bennett LLC, North Haven CT 06473 (US)
(72) Inventor: Vandine, Joseph Douglas, Manteca CA 95337 (US); Karst, Edward F., South Pasadena, California 91030 (US); Baker, Clark R., Newman, California 95360 (US)
(74) Representative: Kinsler, Maureen Catherine

(57) **Abstract**

A system for delivering breathing gas to a patient, the system comprising: a regulator configured to control the fractional inspired oxygen (FiO₂) of the breathing gas delivered to the patient; a sensor configured to determine a blood oxygenation level of the patient; a parameter module configured to determine a ventilation parameter; and a controller configured to automatically adjust the FiO₂ of the breathing gas delivered to the patient based on a variance between the determined blood oxygenation level and a predetermined threshold value and/or the determined ventilation parameter and a predetermined threshold value.

## Description

### TECHNICAL FIELD

The present disclosure relates generally to the Held of medical treatment, e.g., a method for controlling delivery of breathing gas to a patient using multiple ventilation parameters.

### BACKGROUND

Conventional breathing assistance systems typically include a gas delivery system, a patient interface to deliver gas to one or more breathing passages of the patient, and a connection system between the gas delivery system and the patient interface, Such breathing assistance systems may be used, among other reasons, for mechanical ventilation of a patient's lungs and/or treatment of an apnea or other medical condition. The gas delivery system may include a controller configured to vary the flow rate, pressure, and/or other characteristics of the gas delivered to the patient, based on the intended treatment or condition of the patient.

Many modem ventilators include sensors operable to measure various parameters regarding the patient's breathing patterns and/or the operation of the ventilator, and may allow the caregiver to adjust ventilator settings to select or adjust the ventilation strategy being implemented. For example, a gas delivery system may sense one or more of the following parameters: airway pressure, exhaled volume, ventilation mode, type of breath, mean airway pressure, peak airway pressure, PEEP/CPAP pressure, plateau pressure, respiratory rate, I:E ratio, tidal volume, minute volume, and spontaneous minute volume.

Clinical treatment of a ventilated patient often requires that physiological characteristics of the patient be monitored to detect the effects of a particular ventilation strategy on a patient or changes in the patient's breathing patterns. Saturation of Peripheral Oxygen (Sp_{O2}) is an estimation of the oxygen saturation level in a patient's blood usually measured with a pulse oximeter and is one physiological characteristic that may be used in ventilation control. For example, a gas delivery system may control the fraction of inspired oxygen (FiO₂) in the gas delivered based on the Sp_{O2} measured. One embodiment employing this example includes a controller that may adjust the FiO₂ by an amount proportional to the difference between a measured Sp_{O2} and a user-specified target Sp_{O2}.

### SUMMARY

In accordance with one embodiment of the present disclosure, a method for controlling the delivery of a breathing gas to a patient is provided. The method may include regulating the fractional inspired oxygen (FiO₂) of the breathing gas delivered to the patient, determining a blood oxygenation level of the patient, determining a ventilation parameter, and automatically adjusting the FiO₂ of the breathing gas delivered to the patient based on the determined blood oxygenation level and the determined ventilation parameter.

In accordance with another embodiment of the present disclosure, a system for delivering breathing gas to a patient is provided. The system may include a regulator configured to control the FiO₂ of the breathing gas delivered to the patient, a sensor configured to determine a blood oxygenation level of the patient, a parameter module configured to determine a ventilation parameter, and a controller configured to automatically adjust the FiO₂ of the breathing gas delivered to the patient based on the determined blood oxygenation level and the determined ventilation parameter.

In accordance with another embodiment of the present disclosure, a tangible computer-readable storage medium may store a set of instructions executable on a processor. The set of instructions may include instructions for regulating the FiO₂ of the breathing gas delivered to the patient, instructions for determining a blood oxygenation level of the patient, instructions for determining a ventilation parameter, and instructions for automatically adjusting the FiO₂ of the breathing gas delivered to the patient based on the determined blood oxygenation level and the determined ventilation parameter

In accordance with another embodiment of the present disclosure, a breathing assistance system may include: means for regulating the fractional inspired oxygen (FiO₂) of the breathing gas delivered to the patient; means for determining a blood oxygenation level of the patient; means for determining a ventilation parameter; and means for automatically adjusting the FiO₂ of the breathing gas delivered to the patient based on the determined blood oxygenation level and the determined ventilation parameter.

### BRIEF DESCRIPTION OF THE DRAWINGS

Some embodiments of the disclosure may be understood by referring, in part, to the following description and the accompanying drawings wherein:

FIGURE 1 illustrates an example breathing assistance system, according to one embodiment of the present disclosure;

FIGURE 2 illustrates an example display device displaying a group of ventilation parameters, according to one example embodiment of the present disclosure;

FIGURE 3 is a flowchart illustrating a method for controlling the delivery of a breathing gas to a patient using multiple ventilation parameters, according to one embodiment of the present disclosure;

FIGURE 4 is a flowchart illustrating a method for controlling the delivery of a breathing gas to a patient including selecting the target value for one or more ventilation parameters in a breathing assistance device, according to another embodiment of the present disclosure; and

FIGURE 5 is a flowchart illustrating a method for controlling the delivery of a breathing gas to a patient including automatic lung recruitment maneuvers, according to another embodiment of the present disclosure.

### DETAILED DESCRIPTION OF THE DRAWINGS

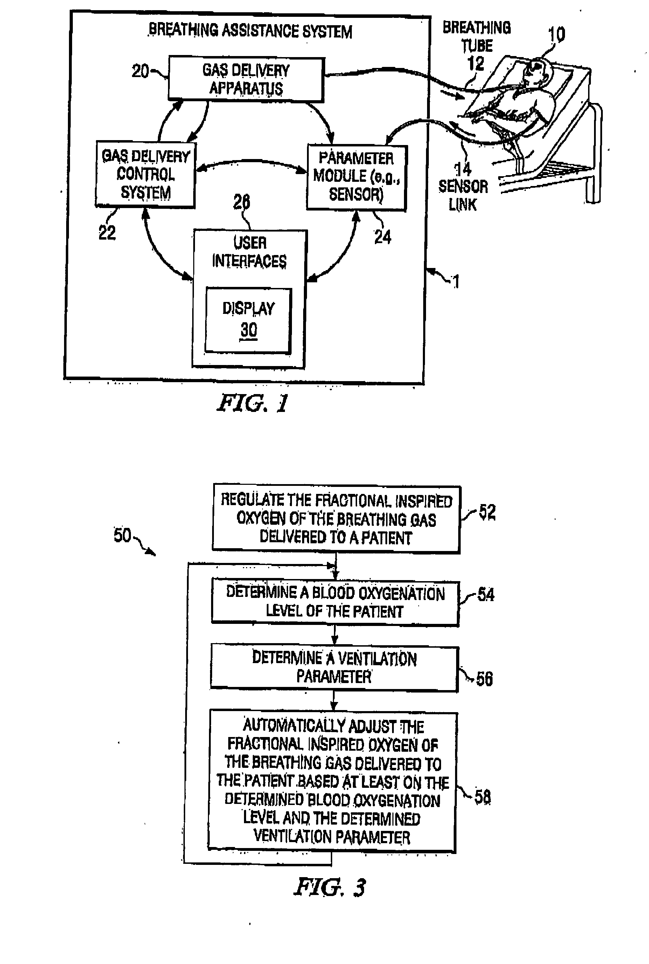

Selected embodiments of the disclosure may be understood by reference, in part, to FIGURES 1-5, wherein like numbers refer to same and like parts. The present disclosure is broadly concerned with breathing assistance systems (e.g., ventilators, CPAP systems, or BiPAP systems) adapted to provide breathing assistance to a patient (e.g., providing ventilation and/or creating an apnea or other breathing condition).

In some embodiments, the breathing assistance system may control breathing assistance based on two or more ventilation parameters. For example, the breathing assistance system may control FiO₂ of the breathing gas delivered based on a measured blood oxygenation level (e.g., SpO₂) and one or more other ventilation parameters.

In other embodiments, the breathing assistance system may control breathing assistance based on an adjustable target value for one or more ventilation parameters. For example, the breathing assistance system may control FiO₂ of the breathing gas delivered based on a target value for blood oxygenation level and may adjust that target value based on one or more other ventilation parameters.

In yet other embodiments, the breathing assistance system may control breathing assistance including automatically recommending and/or initiating lung recruitment maneuvers. For example, the breathing assistance system may control FiO₂ of the breathing gas delivered based on a target value for blood oxygenation level and may recommend a lung recruitment maneuver based on one or more other ventilation parameters.

Ventilation parameters may include any data relevant and/or related to the delivery of breathing gas to a patient. For example, ventilation parameters may include physiological parameters of the patient, medical history of the patient, equipment-related parameters, historical data related to the delivery of breathing gas, and/or user inputs.

Physiological parameters of the patient may include any ventilation parameter measured, sensed, and/or related to the patient's condition (e.g., blood oxygenation level (e.g., SpO₂), lung compliance, lung resistance, rapid-shallow breathing index (RSBI), patient work of breathing (WOB), pulse rate, blood pressure, temperature, and/or any other indicator of the patient's condition). As examples, some physiological parameters may be sensed by the breathing assistance system and/or gathered from a user.

Medical history of the patient may include any ventilation parameter related to the historical condition of the patient (e.g., age, height, weight, medication, historical blood pressure, previous and/or current disease, and/or any other information that might be used to inform and/or adjust medical treatment of the patient). As examples, ventilation parameters related to a patient's medical history may be gathered from a user, delivered to the breathing assistance system from an information handling system (e.g., a computer, a doctor's PDA, etc.), and/or received from any other source of information for a patient's medical history.

Equipment related parameters may include any ventilation parameter related to the operation of the breathing assistance system (e.g., signal quality of a physiological parameter, Positive end-expiratory pressure (PEEP), set PEEP, PEEP_{HIGH}, PEEP_{LOW}, peak inspiratory pressure, tidal volume, set tidal volume, plateau pressure, frequency, pressure support, volume support, percent support, total work of breathing, airway pressure, exhaled volume, ventilation mode, type of breath, mean airway pressure, peak airway pressure, plateau pressure, respiratory rate, I:E ratio, tidal volume, minute volume, spontaneous minute volume, end expiratory flow, inspiratory time, and/or any other ventilation parameter related to the operation of the breathing assistance system). As examples, equipment-related parameters may include data sensed and/or measured by the breathing assistance system, historical data related to the control and/or operation of the breathing assistance system, and/or any other data related to the operation of the breathing assistance system.

Any one or more ventilation parameters may be displayed on a display device. As examples, a display device may display an instantaneous value for any ventilation parameter, a trace or historical trend for any ventilation parameter, an alarm or alert if the value of a ventilation parameter passes a pre-set or calculated threshold, and/or any other indication related to a ventilation parameter that might be of use to a clinician, the patient, and/or other user of a breathing assistance system.

FIGURE 1 illustrates an example breathing assistance system 1 for providing breathing assistance to a patient 10, according to one embodiment of the disclosure. Breathing assistance system 1 may be generally configured to provide breathing assistance to a patient 10 (e.g., to provide ventilation and/or treat an apnea, snoring, or other breathing condition). Breathing assistance system 1 may include a gas delivery apparatus 20, a gas delivery control system 22 for regulating the delivery of breathing gas to patient 10, a parameter module 24 for collecting and/or manipulating data related to ventilation parameter(s), one or more user interfaces 26 for receiving user input, and a display 30 for displaying ventilation parameters and/or other data related to breathing assistance system 1.

Patient 10 may be connected to breathing assistance system 1 by a variety of devices and/or components. For example, breathing gas may be delivered toward a patient through a patient circuit 12. Patient circuit 12 may include tubes, conduits, breathing masks, endotracheal tubes, and/or any other component or device configured for the delivery of gas toward patient 10. As another example, breathing assistance system 1 may receive data related to patient 10 through link 14. In some embodiments, link 14 may be configured to communicate data gathered from patient 10 by one or more sensors (e.g., a pulse-oximeter sensor, a blood-pressure cuff, and/or any other physiologic parameter that might be of use in the treatment of patient 10).

Gas delivery apparatus 20 may include any device or devices configured to generate, supply, and/or deliver gas (e.g., pressurized air) toward patient 10 via a connection system (e.g., a breathing circuit) and/or a patient interface (e.g., a tracheal tube or mask). For example, gas delivery apparatus 20 may comprise a device capable of generating pressurized air (e.g., a ventilator, CPAP system, or BiPAP system), a wall outlet through which pressurized air may be supplied (e.g., in a hospital or clinic), one or more tanks of compressed gas, a compressor, an oxygen concentrator, an oxygen reservoir, an oxygen conserver, or any other suitable source of pressurized or non-pressurized gas. Gas delivery apparatus 20 may further include any other components suitable for providing functionality related to providing breathing assistance to a patient 10. For example, gas delivery apparatus 20 may include one or more sensors, a humidifier, a nobulizer, an alarm system, and/or any other suitable components.

As used herein, the term "gas" may refer to any one or more gases and/or vaporized substances suitable to be delivered to and/or from a patient via one or more breathing orifices (e.g., the nose and/or mouth), such as air, nitrogen, oxygen, any other component of air, CO₂, vaporized water, vaporized medicines, and/or any combination of two or more of the above, for example.

As used herein, the term "patient" may refer to any person or animal that may receive breathing assistance from system 1, regardless of the medical status, official patient status, physical location, or any other characteristic of the person. Thus, for example, patients may include persons under official medical care (e.g., hospital patients), persons not under official medical care, persons receiving care at a medical care facility, persons receiving home care, etc.

Gas delivery control system 22 may be operable to control the breathing assistance provided by gas delivery apparatus 20 based on various input. For example, gas delivery control system 22 may regulate the pressure and/or flow of gas delivered to patient 10 based on various input received by gas delivery control system 22. Such input may include input received from an operator (e.g., via a touch screen and/or other user interfaces 26), data related to the operation of breathing assistance system 1, and/or data received by parameter module 24 via link 14 (e.g., one or more sensors or other electronic devices), Gas delivery control system 22 may be configured to deliver gas based on any protocol and/or treatment regimen useful in the care of patient 10. For example, gas delivery control system may be configured to perform lung recruitment maneuvers.

Gas delivery control system 22 may include, or have access to, one or more processors, memory devices, and any other suitable hardware or software. The one or more memory devices may store instructions (e.g., any suitable software, algorithms, or other logic or instructions that may be executed by one or more processors) for controlling the operation of gas delivery apparatus 20, e.g., controlling ventilation support provided by gas delivery apparatus 20. As another example, gas delivery control system 22 may include any system or device for executing code or logic instructions (e.g., software or firmware) for controlling user interface 26, e.g., a microcontroller, a digital signal processor (DSP), an application specific integrated circuit (ASIC), electrically-programmable read-only memory (EPROM), or a field-programmable gate array (FPGA).

Parameter module 24 may include a sensor, a storage device, and/or any other device or component configured to collect and/or to manipulate data related to one or more ventilation parameters. For example, parameter module 24 may include a storage device to store discrete values for ventilation parameter and/or historical trends of ventilation parameters (e.g., electrically-programmable read-only memory (EPROM), a field-programmable gate array (FPGA), a computer disk, a flash drive, punch cards, Random Access Memory (RAM), or any other system suitable for such storage). As another example, parameter module 24 may include one or more sensors configured to collect ventilation parameters, such as sensors associated with patient 10 (e.g., a pulse-oximeter sensor and monitor), gas delivery apparatus 20, the surrounding atmosphere, and/or any other data related to patient 10 and/or the operation of gas delivery apparatus 20. As yet another example, parameter module 24 may be configured to receive data from a user via user interface 26 (e.g., a keyboard, a touchpad, and/or any other device described below).

User interface 26 may include any systems or devices allowing a user to input data or otherwise interact with breathing assistance system 1, as well as any systems or devices allowing data output to a user. For example, user interface 26 may include one or more physical interfaces (e.g., physical buttons, knobs, sliders, dials, levers, or switches) provided by breathing assistance system 1. As another example, user interface 26 may be provided by a graphical user interface (GUI), and/or a touch screen display (e.g., on display 30). User interface 26 may be coupled to, integrated with, or otherwise associated with display 30, gas delivery control system 22, and/or gas delivery apparatus 20. As examples, user interface 26 may include electrical interfaces (e.g., a desktop computer, laptop computer, or network terminal), and/or other means of interface (e.g., a wireless control pad or a PDA).

In some embodiments, user interface 26 may be operable to exhibit one or more ventilation parameters. For example, in the case of physical user interfaces (e.g., physical buttons, knobs, sliders, dials, levers, or switches), various identifiers may be written or otherwise displayed on the physical user interfaces. As another example, in the case of a touch screen display, various ventilation parameters may be displayed on the screen.

As discussed above, user interface 26 may include or be associated with display 30 configured to display various information regarding breathing assistance system 1 (e.g., data regarding patient 10, the operation of gas delivery apparatus 20, menus, icons, selection tools and/or any other relevant data). In particular, display 30 may display the ventilation parameters selected by breathing assistance system 1 or by a user for display. Display 30 may indicate a trend for particular ventilation parameters by displaying a plot of each ventilation parameter versus time. Display 30 may indicate one or more ventilation parameters in any other suitable manner to a user (e.g., using any suitable graphics, text, numerical values, colors, or other indications).

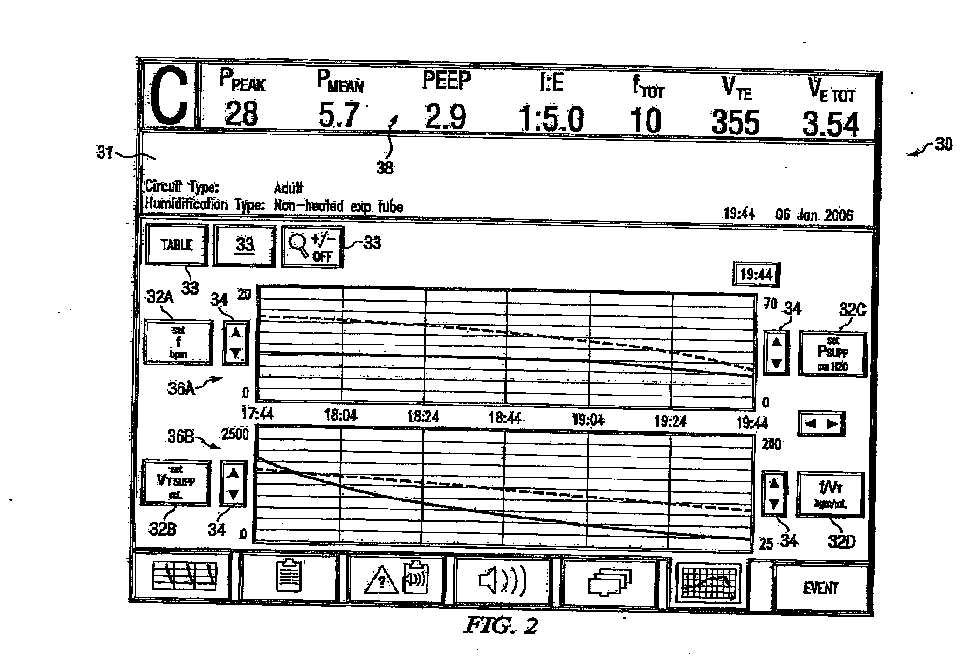

FIGURE 2 depicts an example display 30 displaying a group of ventilation parameters, according to one example embodiment of the present disclosure. Display 30 may include any system or device for displaying various information regarding breathing assistance system 1 (e.g., data regarding patient 10, the operation of gas delivery apparatus 20, menus, icons, selection tools and/or any other relevant data). Display 30 may comprise any type of screen or other visual display (e.g., a touch screen display, CRT, LCD, and/or oscilloscope). Display 30 may be partially or fully integrated with, or may be physically separate from, gas delivery apparatus 20. For example, display 30 may comprise an integrated screen of a ventilator, CPAP, or BiPAP device, or a separate device such as a stand-alone monitoring device or a laptop computer. As discussed above, display 30 may display various ventilation parameters related to breathing assistance system 1 (e.g., data regarding patient I, the operation of gas delivery apparatus 20, menus, icons, selection tools, and/or any other relevant data).

The ventilation parameters and/or other data displayed by display 30 may be determined in any suitable manner and based on any suitable data or input. For example, one or more preprogrammed sets of data and/or ventilation parameters may be selected by a user. As another example, in some embodiments, display 30 may be configured and/or modified by a user (e.g., a technician or caregiver). In such embodiments, user interface 26 may allow a user to configure/modify one or more of the ventilation parameters displayed by display 30 (e.g., via a series of menus, prompts, and/or other user input systems).

In the illustrated embodiment, display 30 includes a touch screen GUI display 31 which displays a number of selectable buttons 33, which operate as user interfaces, e.g., for navigating through screens or displays and/or for selecting, configuring, and/or modifying various ventilation parameters regarding breathing assistance system 1. In the illustrated embodiment, a user may use buttons 33 to display and/or select one or more ventilation parameters for display. For example, as shown in FIGURE 2, display 30 may display a set of tags 32 that each identify a particular ventilation parameter. When the user selects a particular ventilation parameter for display (using buttons 33 or other user interface 26), the selected ventilation parameters may be displayed (e.g., simultaneously or otherwise) in display 30. As discussed above, a value for each ventilation parameter may be displayed in any suitable manner to indicate a trend for that ventilation parameter over time, an instantaneous value for the ventilation parameter, and/or any other information related to one or more ventilation parameters.

As one example, display 30 may show an instantaneous value for one or more ventilation parameters in banner 38. As another example, a plot of multiple ventilation parameters over time (e.g., f, V_{T SUPP}, P_{SUPP}), and f/V_{T}) may be displayed, as shown in charting areas 36A and 36B. In this embodiment, each charting area 32 may be used to display a pair of ventilation parameters (e.g., 32A and 32B). Data for ventilation parameters may be distinguished from each other by color, line type, or in any other manner. In other embodiments, each ventilation parameter may have a separate charting area 32. As shown in FIGURE 2, display 30 may also include scale adjusters 34 that may be selected by a user to adjust the scale for the relevant data, e.g., to keep the trend data from moving outside of the relevant charting area (e.g., when a value becomes too low or too high).

### Controlling the FiO₂ of the breathing gas delivered to the patient based on more than one ventilation parameter

In accordance with certain embodiments of the present disclosure, breathing assistance system 1 may control the fraction of inspired oxygen (FiO₂) of the breathing gas delivered to patient 10 based on more than one ventilation parameter. For example, breathing assistance system 1 may primarily control FiO in the delivered breathing gas based on the measured arterial blood oxygenation level (e.g., SpO₂, and/or PaO₂) and one or more additional ventilation parameters.

The following examples are offered as particular embodiments to illustrate the selection of the one or more additional ventilation parameters and are not intended to be an exhaustive list. For example, breathing assistance system 1 may control FiO₂ of the breathing gas delivered to patient 10 based on measured SpO₂ and at least one additional physiological parameter (e.g., pulse rate, respiration rate, minute volume, tidal volume, inspiratory pressure, blood pressure, cardiac output, rapid shallow breathing index (RSBI), etc.). In another example, breathing assistance system 1 may use at least one equipment-related parameter (e.g., one or more signal quality parameters associated with the indication of blood oxygenation). In another example, breathing assistance system 1 may use at least one user input. In another example, breathing assistance system 1 may use at least one historical datum stored in parameter module 24 (e.g., the number of previous adjustments to FiO₂ in the last thirty minutes), In each example, the data used to control the delivery of breathing gas to patient 10 may be collected by parameter module 24 using link 14 or any other device or component for collecting data.

In accordance with the teachings of the present disclosure, gas delivery control system 22 may combine a measured value of blood oxygenation (e.g., SpO₂, and/or pO₂) and at least one additional ventilation parameter in any useful manner to control FiO₂ of the breathing gas delivered to patient 10. The combination may be any algorithm and/or mathematical function that provides a clinically useful indication related to the delivery of breathing gas to patient 10. For example, gas delivery control system 22 may establish asymmetric criteria for adjusting FiO₂ in one direction (e.g., inhibiting any decrease of FiO₂ if the patient's pulse rate has deviated from a programmed baseline but allowing increases of FiO₂ without regard to the patient's pulse rate). In another embodiment, gas delivery control system 22 may adjust the FiO₂ by an amount proportional to the difference between a measured SpO₂ and a user-specified target value for SpO₂. As a secondary consideration, however, breathing assistance system 1 may consider the prior adjustment of FiO₂ and delay adjusting FiO₂ a second time until some time has passed (e.g., FiO₂ can only be changed once in five minutes). Although the following paragraphs offer specific examples of such control schemes, they are not exhaustive. Persons having ordinary skill in the art will be able to use the teachings of the present disclosure to their advantage in other specific embodiments.

In some embodiments, gas delivery control system 22 may adjust FiO₂ with a goal of maintaining a target value of blood oxygenation for patient 10. Using teachings of the present disclosure, gas delivery control system 22 may adjust the target value of oxygenation based on one or more additional ventilation parameters. For example, gas delivery control system 22 may primarily adjust FiO₂ in order to match measured SpO₂ to a target SpO₂ value (e.g., a target SpO₂ value set by a clinician) and secondarily adjust the target SpO₂ value in response to a change in one or more additional ventilation parameters. For example, gas delivery control system 22 may reduce the target SpO₂ value if the patient's blood pressure reduces below a defined threshold value. In another example, gas delivery control system 22 may increase the target SpO₂ value if the patient's pulse rate or respiratory rate increases above a defined threshold value.

Gas delivery control system 22 may change the magnitude, interval, and/or response time of such adjustment based on one or more additional ventilation parameters. In another example, gas delivery control system 22 may inhibit the adjustment of the target SpO₂ value based on one or more ventilation parameters related to the condition of the equipment used for FiO₂ control or delivery (e.g., gas delivery apparatus 20 and gas delivery control system 22). For example, gas delivery control system 22 may delay the adjustment of FiO₂ if the supply of oxygen for use by gas delivery control system 22 is below a certain threshold value and/or is being replaced.

In some embodiments, gas delivery control system 22 may be configured to provide a user alert and/or another indication to a user and/or clinician, For example, some criteria for an alert may include the FiO₂ of the delivered breathing gas, the measured value of blood oxygenation, and one or more additional ventilation parameters. For example, gas delivery control system 22 may be configured to provide an alert if adjustment of FiO₂ of the breathing gas delivered to patient 10 has been inhibited due to the value of a second ventilation parameter (e.g., pulse rate). As another example, gas delivery control system 22 may be configured to provide an alert if the FiO₂ of the breathing gas increases to or above a defined threshold without an accompanying increase in measured SpO₂ to the SpO₂ target level (e.g., an alert for possible change in the condition of the patients lungs).

Gas delivery control system 22 may be configured to provide an audible alarm, a visual warning (e.g., a flashing light or a text display), and/or a tactile indicator (e.g., vibrating a PDA worn by a clinician), using display 30 and/or user interface 26. Additional examples include embodiments using display 30 as shown in FIGURE 2. Ventilation parameters displayed in charting area 36 may include an alert level or line (e.g., showing an upper or lower limit). Charting area 36 may include a vertical demarcation indicating specific times when an adjustment has been made. Display 30 may also show a trace or trend of adjustments to FiO₂ over time along with any data that underlie the adjustments.

As a specific embodiment, gas delivery control system 22 may inhibit decreases in FiO₂ of the breathing gas delivered to patient 10 when any of the following conditions is true: (a) FiO₂ of the breathing gas delivered to patient 10 has already dropped by a predetermined amount within a predetermined time (e.g., 5 percent in the last 5 minutes); (b) status information from a pulse oximeter indicates that the underlying oximetry signal may be of poor quality (e.g., the oximeter's Pulse Search indication is set); or (c) the current pulse rate, respiration rate, and minute volume have changed by a predetermined amount from a previously measured baseline (e.g., 25 percent of the baseline pulse rate or respiration rate or 30 percent of the baseline minute volume since starting the SpO₂- targeted FiO₂ controller). These asymmetric criteria for FiO₂ adjustment may implement a fast-increase, slow-decrease behavior for FiO₂, which is desirable to mitigate the acute risks of hypoxia as well as the chronic risks of hyperoxia.

In another specific embodiment, gas delivery control system 22 may be configured to inhibit any adjustments in FiO₂ of the breathing gas delivered to patient 10 if any of the following conditions is true: (a) an oximetry signal is not available; (b) the ventilator cannot supply the requested FiO₂ of the breathing gas delivered to patient 10 (e.g., there is a loss of gas pressure in the supply lines, or the patient circuit is occluded or disconnected); or (c) the user has provided an override (e.g., selected a specific FiO₂ of the breathing gas delivered to patient 10 for a predetermined time period, such as 100% for two minutes during suctioning, or 21% for 30 minutes for a weaning trial).

As another example, user interface 26 may be configured to accept user settings for high and low limits on FiO₂ of the breathing gas delivered to patient 10 that would prevent gas delivery control system 22 from adjusting FiO₂ of the breathing gas delivered to patient 10 beyond those limits. The teachings of the present disclosure may provide enhanced safety features in comparison to traditional controller for gas delivery systems (e.g., reducing the risk of hypoxia resulting from reduced FiO₂ of the breathing gas delivered to patient 10 and/or reducing the risk of oxygen toxicity resulting from excess FiO₂ of the breathing gas delivered to patient 10 over the long term). The teachings of the present disclosure may be used to control delivery of breathing gas to a patient based on any combination of ventilation parameters and may provide these or additional benefits.

FIGURE 3 is a flowchart illustrating a method 50 for controlling the delivery of a breathing gas to a patient based on multiple ventilation parameters in accordance with teachings of the present disclosure. Method 50 may include any steps appropriate for controlling the delivery of breathing gas to a patient based on any two or more ventilation parameters.

At Step 52, breathing assistance system 1 may regulate the FiO₂ of the breathing gas delivered to patient 10. For example, gas delivery control system 22 may regulate the FiO₂ of the breathing gas delivered to patient 10 by controlling the amount of oxygen let into the stream of breathing gas, e.g., by controlling one or more valves, regulating the pressure, and/or regulating the flow rate of oxygen.

At Step 54, breathing assistance system 1 may determine a blood oxygenation level of patient 10. Breathing assistance system 1 may determine a blood oxygenation level of patient 10, for example, by sensing the patient's SpO₂ or any other measurement indicating a blood oxygenation level. As another example, breathing assistance system 1 may receive data from a sensor, a parameter module, a user, and/or any other method for determining a blood oxygenation level.

At Step 56, breathing assistance system 1 may determine another ventilation parameter. As discussed above, a ventilation parameter may include any data relevant and/or related to the delivery of breathing gas to a patient. For example, ventilation parameters may include physiological parameters of the patient, medical history of the patient, equipment-related parameters, historical data related to the delivery of breathing gas, and/or user inputs.

In addition, breathing assistance system 1 may determine another ventilation parameter in any appropriate manner. As an example, breathing assistance system 1 may use sensors configured to collect ventilation parameters, such as sensors associated with patient 10, gas delivery apparatus 20, the surrounding atmosphere, and/or any other data related to patient 10 and/or the operation of gas delivery apparatus 20. As another example, breathing assistance system 1 may be configured to receive data from a user (e.g., a keyboard, a touchpad, and/or any other device described above).

At Step 58, breathing assistance system 1 may automatically adjust the FiO₂ of the breathing gas delivered to patient 10 based on the determined blood oxygenation level and the determined ventilation parameter. For example, gas delivery control system 22 may control FiO₂ of the breathing gas delivered to patient 10 based on measured SpO₂ and at least one physiological parameter, the medical history of the patient, equipment-related parameters, historical data, and/or user inputs.

Breathing assistance system 1 may perform method 50 continuously, periodically, upon a triggering event (e.g., automatically upon detecting a change in one or more particular ventilation parameters), and/or according to any schedule selected by a clinician and/or other user of breathing assistance system 1. For example, breathing assistance system 1 may perform method 50 every 15 seconds, every 5 minutes, every 10 breathing cycles, upon a triggering event (e.g., upon detection of a change in SpO₂ or pulse rate), and/or using a combination of schedule and/or triggering events. Breathing assistance system 1 may communicate data related to method 50 to a clinician and/or user through display 30.

### Selecting Target values in a Breathing Assistance System

In operation, breathing assistance system 1 may control FiO₂ of the breathing gas delivered to patient 10 based at least on target values for one or more ventilation parameters. For example, breathing assistance system 1 may primarily control the fraction of inspired oxygen (FiO₂) in the breathing gas delivered based on a target SpO₂ value (e.g., gas delivery control system 22 may adjust the FiO₂ by an amount proportional to the difference between a measured SpO₂ and a user-specified target SpO₂ value). In accordance with the teaching of the present disclosure, breathing assistance system 1 may include user interface 26 and/or display 30 configured to adjust the target values for the one or more ventilation parameters.

Gas delivery control system 22 may include one or more default values for the target values of one or more ventilation parameters. For example, gas delivery control system 22 may be configured to use a default target value for SpO₂ and/or end-tidal carbon dioxide (EtCO₂), the default target value corresponding to typical values for a healthy human. In some cases, a clinician and/or user of breathing assistance system 1 may prefer to adjust the target values for one or more ventilation parameters. For example, the normal condition of patient 10, however, may vary due to any of several factors (e.g., age, environment, chronic disease, and/or any other relevant health factor). As another example, changes in the patient's disease or treatment course may change the appropriate target value of one or more ventilation parameters.

For example, an infant born prematurely may require a lower SpO₂ level than a healthy adult, at least in part because of the risk of Retinopathy of Prematurity. As another example, a patient with severe Chronic Obstructive Pulmonary Disease (COPD) may have a blunted CO₂ drive and require a higher CO₂ and/or a lower SpO₂ level than normal adults. These examples are not intended to be exhaustive, as persons having ordinary skill in the art will be able to identify additional medical conditions and/or treatment protocols that may be improved by implementation of the teachings of the present disclosure.

In systems incorporating the teachings of the present disclosure, adjustment of such target-based treatment protocols may be improved and/or more efficient User interface 26 and/or display 30 may allow manual adjustment of the target values of one or more ventilation parameters. In some embodiments of the present disclosure, user interface 26 and/or display 30 may allow a user to choose among automated protocols for adjusting a target-based treatment protocol. User interface 26 and/or display 30 may include a touch screen (e.g., a GUI as shown in FIGURE 2) and/or additional components configured to accept user input, as discussed with relation to FIGURE 1. For example, user interface 26 may include any device for receiving a user input, e.g., a physical interface (a knob, button, dial, etc.), or a button or other interface on a touch screen GUI. In one embodiment, one or more buttons 33 may include a trigger to set the target values for one or more ventilation parameters to a new value (see FIGURE 2).

Such changes may be based on clinical assessments of patient 10, historical data related to one or more ventilation parameters, and/or other data related to the condition and/or treatment of patient 10. In some embodiments, breathing assistance system 1 may recommend adjusted target values for one or more ventilation parameters. In other embodiments, breathing assistance system I may automatically adjust target values for one or more ventilation parameters. In still other embodiments, breathing assistance system 1 may respond to a user input or trigger to begin a process to adjust target values for one or more ventilation parameters. Some examples of these embodiments are discussed below.

In one embodiment, breathing assistance system 1 may include a selector that sets a target value for a ventilation parameter to the current measured value for that ventilation parameter. The current measured value of a ventilation parameter may be determined in any manner, as more fully discussed with relation to FIGURE 1. As one example, user interface 26 may include a selector that sets the target SpO₂ value to the current measured SpO₂ value determined by parameter module 24. As another example, user interface 26 may include a selector that updates a recommended user-selectable target SpO₂ value, either on a periodic basis or subsequent to the occurrence of predetermined events (e.g., after completion of a lung recruitment maneuver or a lung aspiration procedure), based on analysis of measured SpO₂ value determined by parameter module 24.

In another embodiment, breathing assistance system 1 may include a selector that sets a target value for a ventilation parameter to a new value based on a mathematical function of recent measured values for that ventilation parameter. For example, gas delivery control system 22 and/or parameter module 24 may apply a mathematical function to current and/or historical measured values for SpO₂ (e.g., average value, maximum values minimum value, and/or any other mathematical function) to determine and/or set a new target SpO₂ value, As another example, gas delivery control system 22 and/or parameter module 24 may apply a mathematical function to the current measured values of two or more ventilation parameters to calculate a target value for one or more ventilation parameters (e.g., SpO₂).

In another embodiment, breathing assistance system 1 may include a selector that automatically sets a target value for a ventilation parameter based on a specific disease and/or severity states. For example, gas delivery control system 22 may be preprogrammed with target values for one or more ventilation parameters that correlate to a specific condition (e.g., COPD, premature birth, pneumonia, emphysema, severe COPD, congestive heart failure (CHF), etc.).

In one embodiment, user interface 26 may include one or more selectors labeled and/or identified with a specific condition and gas delivery control system 22 may set a target value for one or more ventilation parameters if a user selects that specific condition. In another embodiment, breathing assistance system 1 may include a selector that sets a target value for a ventilation parameter to a new value based on recommended values determined by gas delivery control system 22 in conjunction with parameter module 24 and/or another information handling system. For example, parameter module 24 may gather data from a hospital information system and use the gathered data to recommend and/or automatically set target values for one or more ventilation parameters.

In another embodiment, breathing assistance system 1 may include a selector that sets a target value for a ventilation parameter to one or more proposed target values preprogrammed for the course of the patient's treatment. For example, gas delivery control system 22 may be preprogrammed with target values for one or more ventilation parameters that correlate to a treatment protocol (e.g., weaning, pre-operative, operating room, PACU, ICU, etc.). For example, the proposed SpO₂ target value for a patient that is being weaned from the ventilator during recovery from pneumonia might be set at 96%, whereas the proposed SpO₂ target value for the same patient during the earlier acute phase when in the ICU might have been 92%, in order to limit the risk of volutrauma, while the proposed SpO₂ target value for a patient undergoing elective surgery with healthy lungs might be set to 98%. In such embodiments, user interface 26 may include one or more options for selecting a treatment protocol (e.g., buttons, dials, touch-screen buttons 33 as shown in FIG. 2, etc.).

In another embodiment, breathing assistance system 1 may include a selector that allows a user to select or specify automatic adjustments in the target value for a ventilation parameter within a predetermined range wherein the target value varies as a function of one or more ventilation parameters. For example, the oxygen saturation level required to maintain an adequate partial arterial oxygen pressure (PaO₂) decreases with increases in body temperature or arterial pH, due to shifts in the O₂ dissociation curve. Gas delivery control system 22 and/or parameter module 24 may determine a range of appropriate variations in the SpO₂ target value as a function of changes in the patient's core temperature or blood pH level to compensate for these shifts in the oxyhemoglobin dissociation curve.

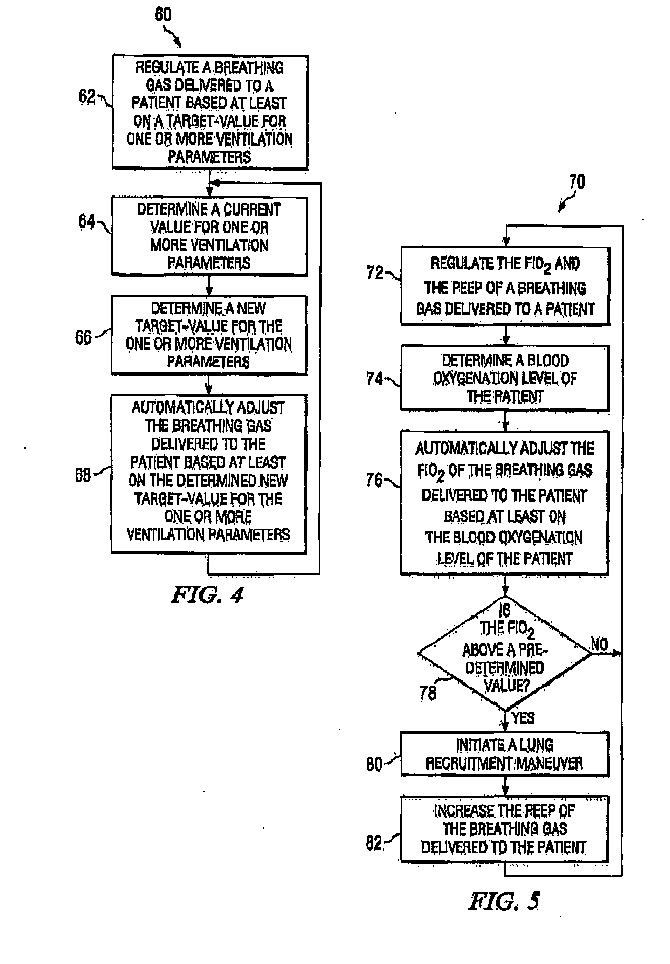

FIGURE 4 is a flowchart illustrating a method 60 for controlling the delivery of a breathing gas to a patient by selecting target values for ventilation parameters in accordance with teachings of the present disclosure. Method 60 may include any steps appropriate for selecting the target value for one or more ventilation parameters in a breathing assistance device. As discussed above, a ventilation parameter may include any data relevant and/or related to the delivery of breathing gas to a patient. For example, ventilation parameters may include physiological parameters of the patient, medical history of the patient, equipment-related parameters, historical data related to the delivery of breathing gas, and/or user inputs.

At Step 62, breathing assistance system 1 may regulate a breathing gas delivered to patient 10 based on a target value for one or more ventilation parameters. For example, gas delivery control system 22 may regulate the pressure and/or flow of gas delivered to patient 10. As another example, gas delivery control system 22 may regulate the FiO₂ of the breathing gas delivered to patient 10 by controlling the amount of oxygen let into the stream of breathing gas. In some embodiments, gas delivery control system 22 may regulate the FiO₂ of the breathing gas delivered to patient 10 based on a target value for the patient's SpO₂.

At Step 64, breathing assistance system 1 may determine a current value for the one or more ventilation parameters (e.g., a blood oxygenation level of patient 10). For example, parameter module 24 may determine a blood oxygenation level of patient 10, for example, by sensing the patient's SpO₂ or any other measurement indicating a blood oxygenation level. As another example, parameter module 24 may receive data from a sensor, a parameter module, a user, and/or any other method for determining a ventilation parameter.

At Step 66, breathing assistance system 1 may determine new target values for the one or more ventilation parameters. For example, breathing assistance system 1 may determine new target values for one or more ventilation parameters based on the analysis of current or historical measured values for the one or more ventilation parameters (e.g., by calculating an average value for one or more ventilation parameters and/or determining a maximum and/or minimum value for the one or more ventilation parameters). As another example, breathing assistance system 1 may determine new target values for one or more ventilation parameters based on the analysis of the patient's disease state, treatment course, and/or medical history.

The determination of new target values for the one or more ventilation parameters may be responsive to a user input, a pre-programmed event, and/or the analysis of one or more ventilation parameters. For example, a user may press one or more buttons 33 associated with display 30 to initiate the determination. As another example, display 30 may display a message, alert, alarm, and/or another indication that a new target value may be appropriate. Display 30 may display a history of prior target values and/or measured values for one or more ventilation parameters and/or any other data that may be useful for a clinician or user to decide whether to change the target values of one or more ventilation parameters.

At Step 68, breathing assistance system 1 may automatically adjust the breathing gas delivered to patient 10 based on the determined new target values for the one or more ventilation parameters. For example, gas delivery control system 22 may control FiO₂ of the breathing gas delivered to patient 10 based on the new determined target value for SpO₂ determined at Step 66.

Breathing assistance system 1 may perform method 60 continuously, periodically, upon a triggering event (e.g., automatically upon detecting a change in one or more particular ventilation parameters), and/or according to any schedule selected by a clinician, and/or other user of breathing assistance system 1, For example, breathing assistance system 1 may perform method 60 every 15 seconds, every 5 minutes, once 10 breathing cycles, upon a triggering event (e.g., detection of a change in SpO₂ or pulse rate), and/or using a combination of schedule and/or triggering events.

### Automatic Lung Recruitment Maneuvers

Lung recruitment maneuvers may be useful in the treatment of patients with certain disease states that may impair oxygen diffusion (e.g., those that involve atalectasis and/or inflammation). For example, patients with pneumonia, respiratory distress syndrome (RDS), acute lung injury (ALI), acute respiratory distress syndrome (ARDS), infant respiratory distress syndrome (IRDS), and/or various injuries to the lung, may exhibit reduced ability to absorb oxygen from air and/or other breathing gas into the lung. In some cases, these conditions are the result of collapsed and/or occluded alveoli in the lung. A lung recruitment maneuver typically consists of providing a breathing gas to patient 10 at a relatively high pressure for enough time to distend collapsed alveoli (e.g., 40 cm water for 40 seconds). Repeated lung recruitment maneuvers, however, may increase the risk of volutrauma and/or barotrauma.

In accordance with teachings of the present disclosure, breathing assistance system 1 may combine controlling the delivery of breathing gas to patient 10 based on one or more ventilation parameters with automatically recommending and/or providing lung recruitment maneuvers. In addition, after a lung recruitment maneuver is completed, breathing assistance system 1 may control PEEP (or another ventilation parameter) to reduce the chance of a new collapse/occlusion and the resulting need for another lung recruitment maneuver.

In one embodiment, breathing assistance system 1 may trigger and/or recommend a lung recruitment maneuver based on a measure of oxygen diffusion across the lung (e.g., the relationship between blood oxygen content (e.g., SpO₂ or PaO₂) and gas oxygen content (e.g., FiO₂ or PAO₂)). In embodiments that regulate the FiO₂ of breathing gas delivered to patient 10 based on a target value for SpO₂, changes in the FiO₂ level may indicate changes in the oxygen diffusion capacity of the patient's lungs, indicating the need for a lung recruitment maneuver. For example, breathing assistance system 1 may trigger and/or recommend a lung recruitment maneuver if the FiO₂ level exceeds a predetermined threshold (e.g., set by breathing assistance system 1 or a clinician). In one specific example, breathing assistance system 1 may trigger a lung recruitment maneuver if patient 10 requires over 60% FiO₂ to maintain SpO₂ over 90%. In some embodiments, gas delivery control system 22 may eliminate, restrict, and/or otherwise inhibit changes in delivery of breathing gas to patient 10 (e.g., inhibit the change of FiO₂ in the breathing gas delivered to patient 10 based on the target value of SpO₂) while the lung recruitment maneuver is underway.

A lung recruitment maneuver may deliver breathing gas at any pressure and/or duration appropriate for the treatment of patient 10. The pressure and/or duration may be default values stored by gas delivery control system 22, user input determined by a clinician and/or another user, and/or determined automatically by delivery control system 22 based on the severity of the patient's condition. These factors may be automatically modified and/or adjusted by gas delivery control system 22 based on one or more ventilation parameters (e.g., time since the last lung recruitment maneuver, rate of recent FiO₂ increases, rate of SpO₂ increases during prior recruitment maneuvers, and/or the extent to which SpO₂ remains below its target value despite FiO₂ increases).

In some embodiments, after performing a lung recruitment maneuver, breathing assistance system 1 may automatically adjust the settings of one or more ventilation parameters related to the delivery of breathing gas (e.g., pressure, flow rate, PEEP, etc.). For example, after performing a lung recruitment maneuver, breathing assistance system 1 may automatically change the target values of one or more ventilation parameters (e.g., SpO₂). In one specific embodiment, breathing assistance system 1 may adjust a PEEP setting for the breathing gas delivered to patient 10 so that post-maneuver PEEP starts at 20 cm water and reduces in 2 cm increments every few minutes as long as SpO₂ remains above 94%. In another specific embodiment, the post-maneuver PEEP may start at a level determined by one or more ventilation parameters (e.g., the time elapsed since the previous recruitment maneuver). Maintaining an elevated PEEP level may maintain lung recruitment longer than returning to the original PEEP level used in the delivery of breathing gas to patient 10, while gradual reductions in PEEP may limit the risk of barotrauma.

After automatically adjusting the settings of the one or more ventilation parameters, breathing assistance system 1 may later automatically adjust the setting of the one or more ventilation parameters consistent with the variation of one or more other ventilation parameters. For example, if breathing assistance system 1 has automatically adjusted the PEEP setting to maintain lung recruitment, it may slowly reduce the PEEP setting going forward. In such embodiments, breathing assistance system 1 may adjust PEEP using a slower response time than adjustments to FiO₂ so that FiO₂ settings may reach equilibrium before any change to the PEEP setting. For example, breathing assistance system 1 may adjust the FiO₂ setting every 30 seconds, if needed, but adjust the PEEP settings every 2-5 minutes, if needed. In addition, breathing assistance system 1 may prevent PEEP adjustments as long as FiO₂ remains above a certain threshold. In another embodiment, breathing assistance system 1 may adjust PEEP settings based on a combination of FiO₂ settings and one or more ventilation parameters (e.g., time elapsed between recruitment maneuvers, pulse rate and/or blood pressure), for example, balancing the benefits of increased alveolar recruitment with reduced central venous return flow.

As another example, breathing assistance system 1 may automatically adjust PEEP both upward and downward after a lung recruitment maneuver. One benefit of bidirectional adjustments may be maintaining FiO₂ at a pre-determined target value that indicates satisfactory oxygen diffusion and remains below the FiO₂ threshold for recommending and/or triggering a subsequent lung recruitment maneuver. The magnitude and/or frequency of such adjustments may be fixed, adjusted by a user and/or clinician, and/or calculated by gas delivery control system 22 based on one or more ventilation parameters. As examples, the adjustments may depend on current FiO₂, the rate of change of FiO₂, and/or the relationship between SpO₂ values and the SpO₂ target In one specific example, if the PEEP level is close to a pre-determined maximum allowed value, downward PEEP adjustments might become larger and/or more frequent than upward adjustments.

FIGURE 5 is a flowchart illustrating a method 70 for controlling the delivery of a breathing gas to a patient, including performing automatic lung recruitment maneuvers, according to one embodiment of the present disclosure. Method 70 may include any steps appropriate for initiating a lung recruitment maneuver based on one or more ventilation parameters, including managing post recruitment PEEP settings to reduce the chance of subsequent lung recruitment maneuvers. As discussed above, a ventilation parameter may include any data relevant and/or related to the delivery of breathing gas to a patient. For example, ventilation parameters may include physiological parameters of the patient, medical history of the patient, equipment-related parameters, historical data related to the delivery of breathing gas, and/or user inputs.

At Step 72, breathing assistance system 1 may regulate the FiO₂ and PEEP of a breathing gas delivered to patient 10. In other embodiments, breathing assistance system 1 may regulate alternative and/or additional ventilation parameters (e.g., inspired pressure, tidal volume, I:B ratio, and/or mandatory breath rate).

At Step 74, breathing assistance system 1 may determine a blood oxygenation level (e.g., SpO₂) of patient 10. Additionally or alternatively, breathing assistance system 1 may determine a value for one or more ventilation parameters (e.g., indications of the diffusion capacity of the patient's lungs). The determination may be performed by parameter module 24 and/or any other components of breathing assistance system 1, e.g., as described with reference to FIGURE 1.

At Step 76, breathing assistance system 1 may automatically adjust the FiO₂ of the breathing gas delivered to patient 10 based on at least the determined blood oxygenation level of patient 10. Additionally or alternatively, breathing assistance system 1 may automatically adjust one or more ventilation parameters (inspired pressure, tidal volume, I:E ratio, and/or mandatory breath rate) based on at least the determined value of one or more ventilation parameters (e.g., pulse and/or blood pressure). For example, breathing assistance system 1 may control FiO₂ of the breathing gas delivered to patient 10 based on the difference between the determined blood oxygenation level of patient 10 and a target value for SpO₂.

At Step 78, breathing assistance system 1 may determine whether the FiO₂ setting is above a predetermined value. In embodiments regulating different and/or additional ventilation parameters, breathing assistance system 1 may determine whether another particular controlled parameter is above or below a pre-determined value. For example, gas delivery control system 22 may determine whether the FiO₂ setting is above 60%. If not, method 70 may return to Step 72.

At Step 80, if breathing assistance system 1 has determined that the FiO₂ setting (or the setting for another controlled ventilation parameter) has crossed a pre-determined threshold, gas delivery control system 22 may initiate a lung recruitment maneuver.

At Step 82, after a lung recruitment maneuver is complete, breathing assistance system 1 may control one or more PEEP settings of the breathing gas delivered to patient 10 to reduce the chances of a subsequent lung recruitment maneuver. Once Step 82 is complete, method 70 may return to Step 72.

Breathing assistance system 1 may perform method 70 continuously, periodically, upon a triggering event (e.g., automatically upon detecting a change in one or more ventilation parameters), and/or according to any schedule selected by a clinician and/or other user of breathing assistance system 1. For example, breathing assistance system I may perform method 60 every 15 seconds, every 5 minutes, once every 10 breathing cycles, upon a triggering event (e.g., each time FiO₂ is adjusted), and/or using a combination of schedule and/or triggering events.

It will be appreciated that while the disclosure is particularly described in the context of breathing assistance systems, the apparatuses, techniques, and methods disclosed herein may be similarly applied in other contexts. Additionally, it should be understood that various changes, substitutions and alterations can be made herein without departing from the spirit and scope of the disclosure as illustrated by the following claims.
The following clauses are included in an form part of the disclosure:
Clause 1. A method for controlling the delivery of a breathing gas to a patient, the method including:
   regulating the fractional inspired oxygen (FiO₂) of the breathing gas delivered to the patient;
   determining a blood oxygenation level of the patient;
   determining a ventilation parameter; and
   automatically adjusting the FiO₂ of the breathing gas delivered to the patient based on the determined blood oxygenation level and the determined ventilation parameter.
Clause 2. The method of clause 1, wherein the blood oxygenation level is determined by measuring the saturation of peripheral oxygen (SpO₂) in the patient.
Clause 3. The method of clause 1, wherein adjusting the FiO₂ of the breathing gas delivered to the patient based on the determined blood oxygenation level and the determined ventilation parameter comprises:
   adjusting the FiO₂ of the breathing gas delivered to the patient to maintain a target value for the blood oxygenation level of the patient; and
   modifying the target value for the blood oxygenation level of the patient based on the determined ventilation parameter.
Clause 4, The method of clause 1, wherein the determined ventilation parameter includes a physiologic characteristic of the patient.
Clause 5. The method of clause 1, wherein the determined ventilation parameter includes one or more patient respiratory parameters.
Clause 6. The method of clause 1, wherein the determined ventilation parameter includes one or more patient cardiovascular parameters.
Clause 7. The method of clause 1, wherein the determined ventilation parameter includes a measurement related to the operation of a gas delivery system.
Clause 8. The method of clause 1, wherein the determined ventilation parameter includes a measurement related to a signal quality associated with either the blood oxygenation level or the determined ventilation parameter.
Clause 9. The method of clause 1, wherein the determined ventilation parameter includes a user input.
Clause 10. The method of clause 1, wherein the determined ventilation parameter includes a history of adjustments to the FiO₂ of the breathing gas delivered to the patient.
Clause 11. The method of clause 1, wherein adjusting the FiO₂ of the breathing gas delivered to the patient based on the determined blood oxygenation level and the determined ventilation parameter includes reducing the magnitude of the adjustment of the FiO₂ of the breathing gas delivered to the patient if the determined ventilation parameter indicates significant variance from pre-determined baseline value.
Clause 12. The method of clause 1, wherein adjusting the FiO₂ of the breathing gas delivered to the patient based on the determined blood oxygenation level and the determined ventilation parameter includes inhibiting the adjustment of the FiO₂ of the breathing gas delivered to the patient if the determined ventilation parameter indicates significant variance from a pre-determined baseline value.
Clause 13. The method of clause 1, wherein adjusting the FiO₂ of the breathing gas delivered to the patient based on the determined blood oxygenation level and the determined ventilation parameter includes changing the frequency of the adjustment of the FiO₂ of the breathing gas delivered to the patient if the determined ventilation parameter indicates significant variance from a pre-determined baseline value.
Clause 14. The method of clause 1, wherein adjusting the FiO₂ of the breathing gas delivered to the patient based on the determined blood oxygenation level and the determined ventilation parameter includes changing the response rate of the adjustment of the FiO₂ of the breathing gas delivered to the patient if the determined ventilation parameter indicates significant variance from a pre-determined baseline value.
Clause 15. The method of clause 1, wherein adjusting the FiO₂ of the breathing gas delivered to the patient based on the determined blood oxygenation level and the determined ventilation parameter includes:
   adjusting the FiO₂ of the breathing gas delivered to the patient based on the determined blood oxygenation level if the determined blood oxygenation level moves in a first direction; and
   adjusting the FiO₂ of the breathing gas delivered to the patient based on the determined blood oxygenation level and the determined ventilation parameter if the determined blood oxygenation level moves in a second direction.
Clause 16. The method of clause 1, further comprising:
   determining a second ventilation parameter; and
   adjusting the FiO₂ of the breathing gas delivered to the patient based on the determined second ventilation parameter.
Clause 17. The method of clause 1, further comprising:
   regulating a second ventilation parameter characteristic of the breathing gas delivered to the patient; and
   automatically adjusting the second ventilation parameter of the breathing gas delivered to the patient based on the determined blood oxygenation level and the determined ventilation parameter.
Clause 18. A system for delivering breathing gas to a patient, the system comprising:
   a regulator configured to control the fractional inspired oxygen (FiO₂) of the breathing gas delivered to the patient;
   a sensor configured to determine a blood oxygenation level of the patient;
   a parameter module configured to determine a ventilation parameter; and
   a controller configured to automatically adjust the FiO₂ of the breathing gas delivered to the patient based on the determined blood oxygenation level and the determined ventilation parameter
Clause 19. The system of clause 18, wherein the sensor determines the saturation of peripheral oxygen (SpO₂) of the patient.
Clause 20. The system clause 18, further comprising the controller configured:
   to automatically adjust the FiO₂ of the breathing gas delivered to the patient to maintain a target value for the blood oxygenation level of the patient; and
   to automatically modify the target value for the blood oxygenation level of the patient based on the determined ventilation parameter.
Clause 21. The system of clause 18, further comprising:
   the parameter module configured to determined a second ventilation parameter; and
   the controller configured to automatically adjust the FiO₂ of the breathing gas delivered to the patient based the second determined ventilation parameter.
Clause 22. The system of clause 18, further comprising:
   the regulator configured to control a second characteristic of the breathing gas delivered to the patient; and
   the controller configured to automatically adjust the second characteristic of the breathing gas delivered to the patient based on the determined blood oxygenation level and the determined ventilation parameter
Clause 23. The system of clause 18, wherein the sensor and the regulator are integrated.
Clause 24. A computer-readable storage medium storing a set of instructions executable on a processor, the set of instructions for controlling the delivery of a breathing gas to a patient, the set of instructions comprising:
   instructions for regulating the fractional inspired oxygen (FiO₂) of the breathing gas delivered to the patient;
   instructions for determining a blood oxygenation level of the patient;
   instructions for determining a ventilation parameter; and
   instructions for automatically adjusting the FiO₂ of the breathing gas delivered to the patient based on the determined blood oxygenation level and the determined ventilation parameter.
Clause 25. A breathing assistance system, comprising:
   means for regulating the fractional inspired oxygen (FiO₂) of the breathing gas delivered to the patient;
   means for determining a blood oxygenation level of the patient;
   means for determining a ventilation parameter; and
   means for automatically adjusting the FiO₂ of the breathing gas delivered to the patient based on the determined blood oxygenation level and the determined ventilation parameter.

## Claims

1. A system for delivering breathing gas to a patient, the system comprising: a regulator configured to control the fractional inspired oxygen (FiO₂) of the breathing gas delivered to the patient; a sensor configured to determine a blood oxygenation level of the patient; a parameter module configured to determine a ventilation parameter; and a controller configured to automatically adjust the FiO₂ of the breathing gas delivered to the patient based on: (1) a variance between the determined blood oxygenation level and a predetermined threshold value and/or (2) a variance between the determined ventilation parameter and a predetermined threshold value.

2. The system of claim 1, wherein the sensor determines the saturation of peripheral oxygen (SpO₂) of the patient.

3. The system claim 1 or claim 2, wherein the controller is configured: to automatically adjust the FiO₂ of the breathing gas delivered to the patient to maintain a target value for the blood oxygenation level of the patient; and to automatically modify the target value for the blood oxygenation level of the patient based on the determined ventilation parameter.

4. The system of any of the preceding claims, wherein the parameter module is configured to determine a second ventilation parameter; and the controller configured to automatically adjust the FiO₂ of the breathing gas delivered to the patient based the second determined ventilation parameter.

5. The system any of the preceding claims, wherein the regulator is configured to control a second characteristic of the breathing gas delivered to the patient; and the controller configured to automatically adjust the second characteristic of the breathing gas delivered to the patient based on the determined blood oxygenation level and the determined ventilation parameter.

6. The system any of the preceding claims, wherein the sensor and the regulator are integrated.

7. The system any of the preceding claims, wherein the controller is configured to automatically adjust the FiO₂ of the breathing gas delivered to the patient based on the determined blood oxygenation level and the determined ventilation parameter by:
adjusting the FiO₂ of the breathing gas delivered to the patient to maintain a target value for the blood oxygenation level of the patient; and modifying the target value for the blood oxygenation level of the patient based on the determined ventilation parameter and/or
reducing the magnitude of the adjustment of the FiO₂ of the breathing gas delivered to the patient and/or
inhibiting the adjustment of the FiO₂ of the breathing gas delivered to the patient if the determined ventilation parameter indicates significant variance from a pre-determined baseline value and/or
changing the frequency of the adjustment of the FiO₂ of the breathing gas delivered to the patient and/or
changing the response rate of the adjustment of the FiO₂ of the breathing gas delivered to the patient if the determined ventilation parameter indicates significant variance from a pre-determined baseline value and/or
adjusting the FiO₂ of the breathing gas delivered to the patient based on the determined blood oxygenation level if the determined blood oxygenation level moves in a first direction; and adjusting the FiO₂ of the breathing gas delivered to the patient based on the determined blood oxygenation level and the determined ventilation parameter if the determined blood oxygenation level moves in a second direction.

8. The system of any of the preceding claims, wherein the determined ventilation parameter includes a physiologic characteristic of the patient and/or one or more patient respiratory parameters and/or one or more patient cardiovascular parameters and/or a measurement related to the operation of a gas delivery system and/or a measurement related to a signal quality associated with either the blood oxygenation level or the determined ventilation parameter and/or a user input and/or a history of adjustments to the FiO₂ of the breathing gas delivered to the patient.

9. The system of claim 1, wherein the parameter module is configured to determine a second ventilation parameter; and the controller is configured to adjust the FiO₂ of the breathing gas delivered to the patient based on the determined second ventilation parameter.

10. The system of claim 1, wherein the regulator is configured to regulate a second ventilation parameter characteristic of the breathing gas delivered to the patient; and automatically adjust the second ventilation parameter of the breathing gas delivered to the patient based on the determined blood oxygenation level and the determined ventilation parameter.

11. A computer-readable storage medium storing a set of instructions executable on a processor, the set of instructions for controlling the delivery of a breathing gas to a patient, the set of instructions comprising: instructions for regulating the fractional inspired oxygen (FiO₂) of the breathing gas delivered to the patient; instructions for determining a blood oxygenation level of the patient; instructions for determining a ventilation parameter; and instructions for automatically adjusting the FiO₂ of the breathing gas delivered to the patient based on: (1) a variance between the determined blood oxygenation level and a predetermined threshold value and/or (2) a variance between the determined ventilation parameter and a predetermined threshold value.

12. A breathing assistance system, comprising: means for regulating the fractional inspired oxygen (FiO₂) of the breathing gas delivered to the patient; means for determining a blood oxygenation level of the patient; means for determining a ventilation parameter, and means for automatically adjusting the FiO₂ of the breathing gas delivered to the patient based on: (1) a variance between the determined blood oxygenation level and a predetermined threshold value and/or (2) a variance between the determined ventilation parameter and a predetermined threshold value.
